# EUROPEAN PATENT APPLICATION

(11) **EP 4 424 674 A1**
(43) Date of publication of application: **04.09.2024**
(21) Application number: 21962506.8
(22) Date of filing: 29.10.2021
(51) Int. Cl.: C07D 237/26, C07D 498/08

(54) **EXTERNAL STIMULUS-RESPONSIVE CLICK REACTION TECHNOLOGY**

(71) Applicant: Tohoku University, Sendai-shi, Miyagi 980-8577 (JP)
(72) Inventor: MIZUKAMI, Shin, Sendai-shi, Miyagi 980-8577 (JP); NOVIANTI, Ira, Sendai-shi, Miyagi 980-8577 (JP); KOWADA, Toshiyuki, Sendai-shi, Miyagi 980-8577 (JP)
(74) Representative: Gill Jennings & Every LLP
(86) International application number: PCT/JP2021/040154
(87) International publication number: WO 2023/073966

(57) **Abstract**

This invention addresses the problem of developing a reaction control method for an inverse electron demand Diels-Alder reaction, and a method for producing a cyclocondensation reaction product utilizing a reactioncontrolled inverse electron demand Diels Alder reaction. This invention provides: a reaction control method for an inverse electron demand Diels-Alder reaction characterized in that reaction control of an inverse electron demand Diels-Alder reaction is performed by using a stimulus response-cleavable macrocyclic tetrazine as the tetrazine in a cyclocondensation reaction of a reaction substrate and tetrazine that utilizes an inverse electron demand Diels-Alder reaction; and a method for producing a cyclocondensation reaction product that utilizes an inverse electron demand Diels-Alder reaction that is reactioncontrolled by this reaction control method.

## Description

### Technical Field

The present invention relates to an external stimulus-responsive click reaction technology. More particularly, the invention is concerned with an external stimulus-responsive click reaction technology in which the reaction can be controlled.

### Background Art

In recent years, a series of reactions called click chemistry, in which a condensation reaction bioorthogonally and specifically proceeds, have attracted great attention. Especially, a cyclocondensation reaction of a transcyclooctene (TCO) and a tetrazine (Tz) or the like is called an inverse electron-demand Diels-Alder (IEDDA) reaction, and has very high reactivity and thus recently has become a key reaction used in various analyses of functions of biomolecules and the like.

Accordingly, freely controlling the reactivity of IEDDA reaction is expected to dramatically expand the range of the application of the reaction. Meanwhile, there are an extremely limited number of reports about studies on the IEDDA reaction control. For example, NPL 1 discloses a technique in which a reaction of tetrazine is controlled by oxidizing a dihydrotetrazine to a tetrazine. However, this method includes an oxidation reaction, and hence has problems in that a background reaction proceeds due to autoxidation of the tetrazine, and in that a response to a stimulus other than oxidation and photooxidation is difficult. Therefore, the development of methodology for freely controlling the reactivity of the IEDDA reaction more stably in response to various stimuli inputs has been desired.

### Citation List

### Non-Patent Literature

NPL 1: Fox et al, J. Am. Chem. Soc. 2016, 138, 18, 5978-5983

### Summary of Invention

### Technical Problem

In view of the above problems, the invention has been made, and the objective of the invention is to develop a reaction control method for an inverse electron-demand Diels-Alder reaction, and a method for producing a cyclocondensation reaction product using an inverse electron-demand Diels-Alder reaction controlled by the reaction control method of the invention.

### Solution to Problem

The present inventors have conducted extensive and intensive studies to solve the above-mentioned problems. As a result, it has been found that when a tetrazine which is one of the reaction substrates is changed to a macrocyclic structure by derivatization, the IEDDA reaction is completely terminated. As a result of studying various structures of tetrazine, it has been found that the IEDDA reaction is terminated in some macrocyclic tetrazines, and further found that when the macrocyclic tetrazine is changed to a noncyclic structure due to a stimulus, the reaction rapidly proceeds. Based on the above findings, the invention has been completed.

Specifically, the invention is directed to the followings.
[1] A method for producing a cyclocondensation reaction product, comprising the step of conducting a cyclocondensation reaction of a reaction substrate and a tetrazine using an inverse electron-demand Diels-Alder reaction,
   in which the reaction control for the inverse electron-demand Diels-Alder reaction is conducted using a stimulus-responsive cleavable macrocyclic tetrazine as the tetrazine.
[2] The production method according to item [1] above, which comprises the following steps a) and b) of:
   a) applying a stimulus to cleave a stimulus-responsive cleavable macrocyclic tetrazine; and
   b) conducting a cyclocondensation reaction of a reaction substrate and the cleaved macrocyclic tetrazine using an inverse electron-demand Diels-Alder reaction.
[3] The production method according to item [1] or [2] above, in which the stimulus-responsive cleavable macrocyclic tetrazine is a compound having a cyclic moiety linking two carbon atoms of the tetrazine, and having a stimulus-responsive cleavable group in the cyclic moiety.
[4] The production method according to any one of items [1] to [3] above, in which the stimulus is selected from pH, a light, an enzyme, an ion, and oxidation-reduction.
[5] The production method according to any one of items [1] to [4] above, in which the tetrazine is 1,2,4,5-tetrazine.
[6] The production method according to any one of items [1] to [5] above, in which the stimulus-responsive cleavable macrocyclic tetrazine is a compound represented by the following formula (I): 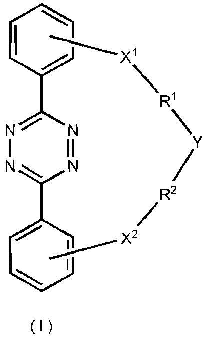
   in which X¹ and X² are a heteroatom or a carbon atom,
   R¹ and R² are independently a single bond, an alkylene, a heteroalkylene, an arylene, a heteroarylene, -CO-, or a combination thereof, and Y is a stimulus-responsive cleavable group.
[7] The production method according to any one of items [1] to [6] above, in which the stimulus-responsive cleavable macrocyclic tetrazine is a compound represented by the following formula: 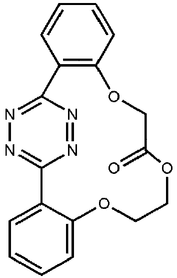
[8] The production method according to any one of items [1] to [7] above, in which the reaction substrate is a compound having a structure selected from a transcyclooctene, a norbornene, and a cyclooctyne.
[9] A reaction control method for an inverse electron-demand Diels-Alder reaction, comprising the step of conducting, in a cyclocondensation reaction of a reaction substrate and a tetrazine using an inverse electron-demand Diels-Alder reaction, the reaction control for the inverse electron-demand Diels-Alder reaction using a stimulus-responsive cleavable macrocyclic tetrazine as the tetrazine.
[10] A compound represented by the following formula (I) : 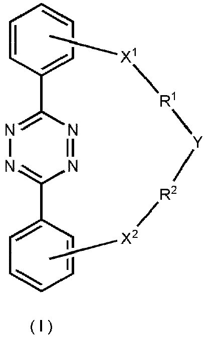
   in which X¹ and X² are a heteroatom or a carbon atom,
   R¹ and R² are independently a single bond, an alkylene, a heteroalkylene, an arylene, a heteroarylene, -CO-, or a combination thereof, and Y is a stimulus-responsive cleavable group.
[11] The compound according to item [10] above, in which the X¹ and X² are oxygen.
[12] The compound according to item [10] or [11] above, in which the R¹ and R² are independently a single bond, an alkylene, an arylene, or a combination thereof.
[13] The compound according to any one of items [10] to [12] above, in which the stimulus-responsive cleavable group is selected from -COO-, -CONH-, -O-, -S-S-, -O-C(CH₃)₂-O-, -O-PO(O-)-O-, and a structure containing a self-immolative group having a structural formula A shown below: 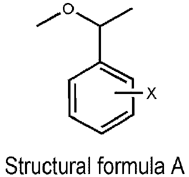 in which X represents a group that is capable of being changed to an electron-donating group.
[14] The compound according to any one of items [10] to [13] above, which is a compound represented by the following formula: 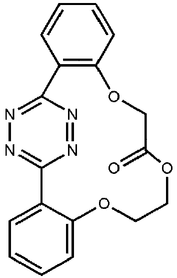
[15] A reagent for an inverse electron-demand Diels-Alder reaction, which comprises the compound according to any one of items [10] to [14] above.

### Advantageous Effects of Invention

In the invention, there is provided a reaction control method for an inverse electron-demand Diels-Alder reaction. In addition, in the invention, there is provided a method for producing a cyclocondensation reaction product using an inverse electron-demand Diels-Alder reaction controlled by the reaction control method of the invention. Further, in the invention, there is provided a macrocyclic tetrazine compound which can be used in the reaction control method of the invention. Further, in the invention, there is provided a reagent for an inverse electron-demand Diels-Alder reaction, which comprises the compound of the invention.

### Brief Description of Drawings

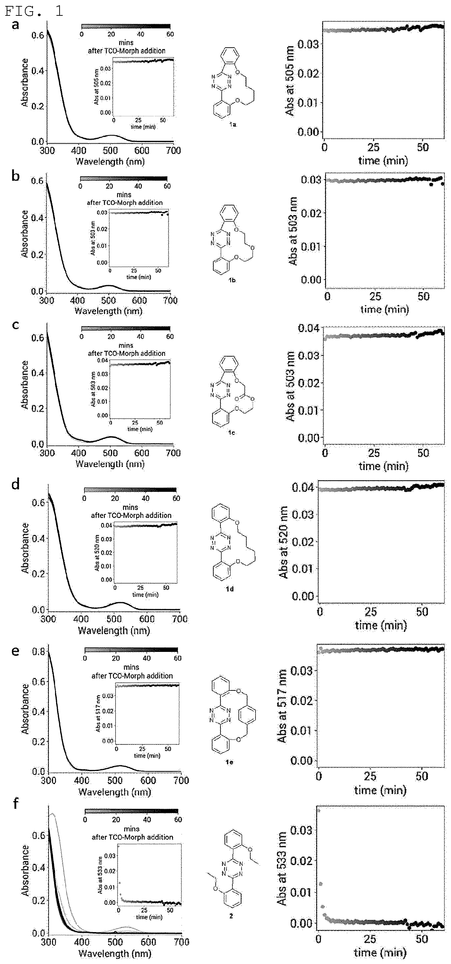

FIG. 1 shows absorption spectra of the synthesized macrocyclic tetrazines and noncyclic tetrazine, and each of the absorption spectral changes by reacting with TCO-Morph. a to f of FIG. 1 show, respectively, the absorption spectra of the macrocyclic tetrazines 1a to 1e and noncyclic tetrazine 2 (before adding TCO-Morph). The graph shown in the right-hand upper position in each graph for spectrum indicates a time-dependent change of the absorption spectrum after adding TCO-Morph. Further, enlarged views of the graphs for time-dependent change are shown on the right.

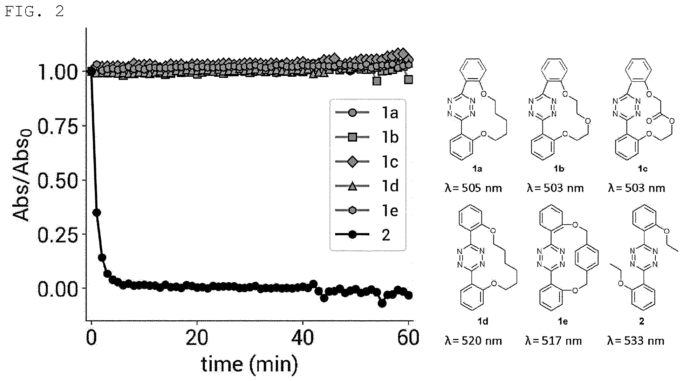

FIG. 2 shows the absorption spectra change of the macrocyclic tetrazines 1a to 1e and noncyclic tetrazine 2 by reacting with TCO-Morph. The absorption maximum wavelengths of the individual compounds are shown on the right.

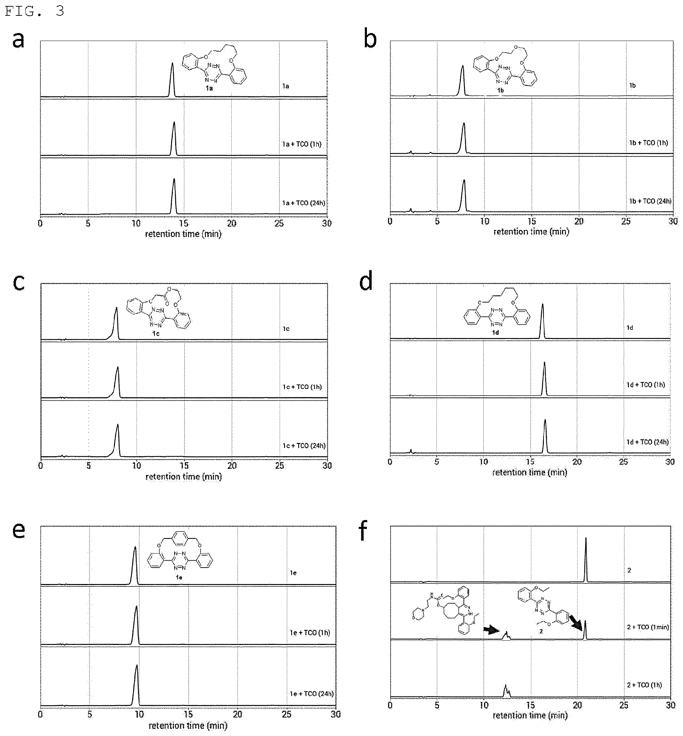

FIG. 3 shows the results of HPLC analysis made with respect to the reaction product of each of the synthesized macrocyclic tetrazines and noncyclic tetrazine with TCO-Morph. a to f of FIG. 3 show, respectively, the results of the macrocyclic tetrazines 1a to 1e and noncyclic tetrazine 2.

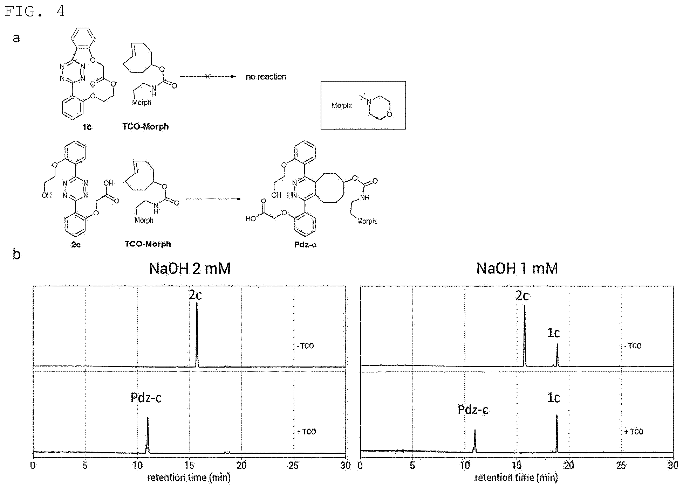

FIG. 4 shows a reaction of the macrocyclic tetrazine 1c and TCO-Morph and the results of HPLC analysis of the reaction product thereof. a of FIG. 4 shows a reaction scheme of the macrocyclic tetrazine 1c or noncyclic tetrazine 2c (cleavage product of the macrocyclic tetrazine 1c) with TCO-Morph. b of FIG. 4 shows the results of HPLC analysis made with respect to the reaction product of the macrocyclic tetrazine 1c with TCO-Morph (in 1:1 MeCN/2 mM aqueous NaOH solution). c of FIG. 4 shows the results of HPLC analysis made with respect to the reaction product of the macrocyclic tetrazine 1c with TCO-Morph (in 1:1 MeCN/1 mM aqueous NaOH solution).

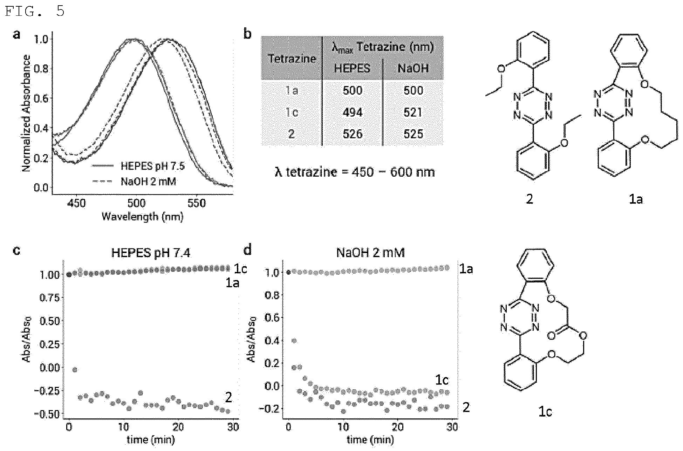

FIG. 5 shows absorption spectra and absorption maximum wavelengths of the macrocyclic tetrazines and noncyclic tetrazine, and a change of the absorption spectra changes by reacting with TCO-Morph. a of FIG. 5 shows absorption spectra (normalized) of the macrocyclic tetrazines and noncyclic tetrazine under neutral conditions and alkaline conditions. b of FIG. 5 shows absorption maximum wavelengths (λₘₐₓ) of the macrocyclic tetrazines and noncyclic tetrazine under neutral conditions and alkaline conditions. c of FIG. 5 shows the time-dependent absorbance changes (normalized) with respect to each of the macrocyclic tetrazines and noncyclic tetrazine under neutral conditions after adding TCO-Morph. d of FIG. 5 shows the time-dependent absorbance changes (normalized) with respect to each of the macrocyclic tetrazines and noncyclic tetrazine under alkaline conditions after adding TCO-Morph.

### Description of Embodiments

Hereinbelow, the invention will be described.

### <Reaction control method for an inverse electron-demand Diels-Alder reaction>

An embodiment of the invention is directed to a reaction control method for an inverse electron-demand Diels-Alder reaction (hereinafter, referred to also as "the reaction control method of the invention"), in which, in a cyclocondensation reaction of a reaction substrate and a tetrazine using an inverse electron-demand Diels-Alder reaction, the reaction control for the inverse electron-demand Diels-Alder reaction is conducted using a stimulus-responsive cleavable macrocyclic tetrazine as the tetrazine.

As mentioned above, the invention has a characteristic feature such that, in a cyclocondensation reaction of a reaction substrate and a tetrazine using an inverse electron-demand Diels-Alder reaction, a macrocyclic tetrazine which is cleavable in response to a stimulus (stimulus-responsive cleavable) is used as the tetrazine. By using the stimulus-responsive cleavable macrocyclic tetrazine, the reaction can be terminated (the tetrazine remains in a nonactive state) under conditions such that a stimulus is not applied. When applying a stimulus that can cleave the stimulus-responsive cleavable macrocyclic tetrazine, the stimulus-responsive cleavable macrocyclic tetrazine is cleaved to form a noncyclic tetrazine. As a result, the tetrazine is activated, making it possible to initiate an inverse electron-demand Diels-Alder reaction. Thus, in the reaction control method of the invention, by controlling the timing, duration, degree, region, and the like for applying a stimulus, the reactivity of the tetrazines can be easily controlled quantitatively and spatiotemporally.

The present inventors have found that the reaction control for the inverse electron-demand Diels-Alder reaction can be conducted by such a method. Based on the above findings, the present inventors have completed the invention.

### <Diels-Alder reaction>

The Diels-Alder reaction is a reaction in which a dienophile is added to a conjugated diene to form a cycloaddition product (reference documents: 1. M. L. Blackman, M. Royzen & J. M. Fox, "Tetrazine Ligation: Fast Bioconjugation Based on Inverse-Electron-Demand Diels-Alder Reactivity", J. Am. Chem. Soc. 130, 13518-13519 (2008).; 2. B. L. Oliveira, Z. Guo & G. J. L. Bernardes, "Inverse electron demand Diels-Alder reactions in chemical biology", Chem. Soc. Rev. 46, 4895-4950 (2017).; 3. E. Kozma, O. Demeter & P. Kele, "Bio-orthogonal Fluorescent Labelling of Biopolymers through Inverse-Electron-Demand Diels-Alder Reactions", ChemBioChem 18, 486-501 (2017).; and 4. T. Reiner & B. M. Zeglis, "The inverse electron demand Diels-Alder click reaction in radiochemistry", J. Label. Compd. Radiopharm. 57, 285-290 (2014).). In the reaction control method of the invention, a reaction can be conducted under substantially the same conditions as known conditions for Diels-Alder reaction, except that a stimulus-responsive cleavable macrocyclic tetrazine is used as a conjugated diene.

Further, the Diels-Alder reaction is a reaction that is caused by an interaction between the highest occupied molecular orbital of a conjugated diene and the lowest unoccupied molecular orbital of a dienophile, but, also when the conjugated diene has a strong electron attractive group and the dienophile has a strong electron-donating group, the reaction is accelerated. In this case, it is considered that an interaction between the lowest unoccupied molecular orbital of the conjugated diene and the highest occupied molecular orbital of the dienophile causes a reaction to proceed, and such a reaction is called an inverse electron-demand Diels-Alder reaction.

With respect to, for example, the molar ratio of the stimulus-responsive cleavable macrocyclic tetrazine and the dienophile used in the Diels-Alder reaction, there is no particular limitation, but the molar ratio of the stimulus-responsive cleavable macrocyclic tetrazine and dienophile is preferably 5:1 to 1:5, more preferably 3:1 to 1:3, further preferably 2:1 to 1:2, especially preferably 1.5:1 to 1:1.5, most preferably equimolar.

In the Diels-Alder reaction, from the viewpoint of improving the reaction efficiency, a solvent may be added to the reaction system. The solvent can be appropriately selected from solvents used in the conventional Diels-Alder reaction, and preferred is a solvent that does not inhibit the reaction and has high compatibility with the stimulus-responsive cleavable macrocyclic tetrazine and dienophile.

Generally, water can be used as a solvent. With respect to the solvent, a liquid other than water may be used, and, for example, an organic solvent can be used. Further, among the organic solvents, an amphiphilic solvent, in other words, an organic solvent miscible with water is preferred. Specific examples of solvents other than water include alcohol solvents, such as methanol, ethanol, and 1-butanol, as well as THF (tetrahydrofuran), DMF (N,N-dimethylformamide), NMP (N-methylpyrrolidone), DMSO (dimethyl sulfoxide), dioxane, acetonitrile, pyridine, acetone, and glycerol.

Further, a salt may be added to the solvent. The type of the salt is arbitrary as long as the effects of the invention are not extremely sacrificed, and specific examples of salts include NaCl, KCl, sodium phosphate, sodium acetate, calcium chloride, sodium hydrogen carbonate, and ammonium carbonate. Further, with respect to the amount of the salt used, there is no particular limitation, and the salt can be used in an arbitrary amount according to the use. Further, salts may be used individually or in combination arbitrarily with an arbitrary ratio.

In addition, when water is used as a solvent, pure water can be used as the water, or an aqueous solution having dissolved therein a solute other than the reaction substrate can be used. Examples of such aqueous solutions include various types of buffers, and specific examples of buffers include a carbonate buffer, a phosphate buffer, an acetate buffer, a HEPES buffer, and a TRIS buffer. Solvents may be used individually or in combination arbitrarily with an arbitrary ratio.

Further, if necessary, an additive may be mixed, in addition to the reaction substrate. Examples of additives include the above-mentioned salts, an acid, a base, a buffer, a humectant such as glycerol, metal ions such as zinc as a stabilizer for biomaterial, an anti-foaming agent, and a modifier. Note that additives may be used individually or in combination arbitrarily with an arbitrary ratio.

The Diels-Alder reaction has such high reactivity that the reaction smoothly proceeds at a relatively low temperature of about 20 to 40°C, so that the reaction is terminated in a practical time of about 1 to 6 hours. When accelerating the reaction, the reaction system may be heated to, for example, about 50 to 100°C.

Further, the reaction smoothly proceeds without a catalyst, but a known catalyst used in a known Diels-Alder reaction may be used.

The reaction control method of the invention comprises, for example, the following steps.
(1) A reaction system containing a stimulus-responsive cleavable macrocyclic tetrazine as a conjugated diene, and a dienophile (hereinafter, these are frequently correctively referred to as "reaction substrate") is prepared.
(2) A stimulus is applied to cleave the stimulus-responsive cleavable macrocyclic tetrazine. In this step, by controlling the timing, duration, region, amount, and the like of applying a stimulus, reaction control for the inverse electron-demand Diels-Alder reaction can be made. With respect to the application of a stimulus, a stimulus may be artificially applied, or a stimulus may be applied due to a change caused in the surrounding environment by a bioreaction or the like.
(3) The cleaved macrocyclic tetrazine (noncyclic tetrazine) is used in an inverse electron-demand Diels-Alder reaction to conduct a cyclocondensation reaction with a reaction substrate. The Diels-Alder reaction has such high reactivity that the reaction smoothly proceeds, and therefore generally, the reaction of the step (3) proceeds simultaneously with cleavage of the stimulus-responsive cleavable macrocyclic tetrazine in the step (2).

The reaction control method of the invention is described below with reference to an embodiment as an example in which the stimulus is a light, but the invention is not limited to this embodiment.

The degree of cleavage of the stimulus (light in this embodiment)-responsive cleavable macrocyclic tetrazine depends on the amount of the light with which the stimulus-responsive cleavable macrocyclic tetrazine is irradiated. The larger the amount of the light with which the stimulus-responsive cleavable macrocyclic tetrazine is irradiated, the larger the amount of the stimulus-responsive cleavable macrocyclic tetrazine disintegrated in the irradiated region, or the larger the amount of the activated tetrazine. Accordingly, by controlling the light irradiation amount by appropriately selecting the intensity of the light for irradiation or the irradiation time, the amount of the activated tetrazine may be controlled.

Further, a light is focused on a part of the region of the reaction system so that only the focus region is irradiated with a satisfactory amount of the light, making it possible to activate the tetrazine only in a specific region.

The timing, duration, region, amount, and the like of irradiation of a light can be strictly controlled. The reaction control method of the invention can precisely control development of the tetrazine activity, and development of the tetrazine activity in a necessary amount (degree) can be achieved in a necessary place at a necessary time.

### <Stimulus-responsive cleavable macrocyclic tetrazine>

With respect to the stimulus-responsive cleavable macrocyclic tetrazine, there is no particular limitation as long as it has a structure in which the macrocyclic tetrazine is maintained and nonactive under conditions such that a stimulus is not applied, and applying a stimulus cleaves the macrocyclic tetrazine to form a noncyclic tetrazine, so that the tetrazine is activated. More specifically, the stimulus-responsive cleavable macrocyclic tetrazine is a compound having a cyclic moiety linking two carbon atoms of the tetrazine, and having a stimulus-responsive cleavable group in the cyclic moiety.

With respect to the tetrazine, there is no particular limitation, and the tetrazine may be 1,2,4,5-tetrazine, 1,2,3,4-tetrazine, or the like, but, from the viewpoint of the reactivity and the like, 1,2,4,5-tetrazine is preferred.

With respect to the stimulus-responsive cleavable group contained in the stimulus-responsive cleavable macrocyclic tetrazine, there is no particular limitation as long as the group has a structure which is cleavable due to a stimulus, and a group can be appropriately selected from known stimulus-responsive cleavable groups and used.

With respect to the structure of the cyclic moiety linking two carbon atoms of the tetrazine, there is no particular limitation as long as the structure does not adversely affect the reaction, and can stably maintain the stimulus-responsive cleavable macrocyclic tetrazine under conditions such that a stimulus is not applied. The stimulus-responsive cleavable group may serve as a cyclic moiety.

In the reaction control method of the invention, a derivative of the stimulus-responsive cleavable macrocyclic tetrazine, which is obtained by introducing a substituent, substance or the like, which does not adversely affect the reaction, other than the tetrazine moiety, the cyclic moiety, and the stimulus-responsive cleavable group, into the stimulus-responsive cleavable macrocyclic tetrazine may be used. An embodiment using such a derivative of the stimulus-responsive cleavable macrocyclic tetrazine is included in "the use of the stimulus-responsive cleavable macrocyclic tetrazine" in the cyclocondensation reaction of a reaction substrate and a tetrazine. Introduction of a substituent, substance or the like, which does not adversely affect the reaction, into the stimulus-responsive cleavable macrocyclic tetrazine can be performed using a known organic synthesis method.

Specifically, as an example of the stimulus-responsive cleavable macrocyclic tetrazine, there can be mentioned a compound represented by the following formula (I):

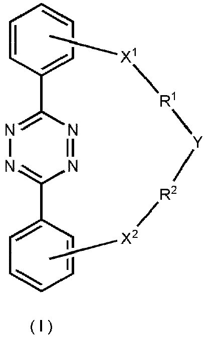

in which X¹ and X² are a heteroatom or a carbon atom, R¹ and R² are independently a single bond, an alkylene, a heteroalkylene, an arylene, a heteroarylene, -CO-, or a combination thereof, and Y is a stimulus-responsive cleavable group.

In the above formula, the phenylene may be any of o-phenylene, m-phenylene, and p-phenylene, and, from the viewpoint of the reactivity and the like, o-phenylene is preferred. The phenylene may have a substituent as long as the substituent does not adversely affect the reaction, and examples of substituents include a halogen, a haloalkyl group, a hydroxyl group, a carboxyl group, an amino group, an alkylamino group, an alkyl group, an alkoxy group, an acyl group, a mercapto group, and an aryl group.

In the above formula, X¹ and X² are a heteroatom or a carbon atom, preferably a heteroatom. The heteroatom is oxygen, nitrogen, sulfur, or the like, preferably oxygen. X¹ and X² are independent of each other, and may be the same atom or different atoms. Further, the heteroatom which X¹ and X² represent has the number of hydrogen atom or atoms according to the atomic species.

In the above formula, R¹ and R² are independently a single bond, an alkylene, a heteroalkylene, an arylene, a heteroarylene, -CO-, or a combination thereof, preferably a single bond, an alkylene, an arylene, or a combination thereof.

In the above formula, Y is a stimulus-responsive cleavable group. With respect to the stimulus-responsive cleavable group, there is no particular limitation as long as it is a group that can be maintained in a nonactive state under conditions such that a stimulus is not applied, and which can be cleaved in response to a stimulus under conditions such that a stimulus is applied, forming a noncyclic tetrazine that is in an active state.

The stimulus-responsive cleavable group may be composed solely of a group that is actually cleavable in response to a stimulus, or may have a structure (atomic group) containing a group that is actually cleavable in response to a stimulus, and a group participating in cleavage or a group having the group participating in cleavage.

When the stimulus-responsive cleavable group has a structure containing a group that is actually cleavable in response to a stimulus, and a group participating in cleavage or a group having the group participating in cleavage, Y and one or two or more groups selected from X¹, X², R¹, R², and phenylene may be combined to form the stimulus-responsive cleavable group. X¹, X², R¹, R², or phenylene may serve as Y.

The stimulus-responsive cleavable group may be appropriately selected according to the stimulus used, and a known stimulus-responsive cleavable group can be used. With respect to the stimulus-responsive cleavable group, reference can be made to, for example, a reference document: F. Maleki et al., Int. J. Pharm. 2019, 572, 118716. With respect to the stimulus-responsive cleavable group, in the stimulus-responsive cleavable macrocyclic tetrazine, one group or two or more groups may be used, and one type of the group may be used, or two types or more of the groups may be used.

With respect to the stimulus-responsive cleavable group, there is no particular limitation, but examples of stimulus-responsive cleavable groups include -COO-, -CONH-, -O-, -S-S-, -O-C(CH₃)₂-O-, -O-PO(O-)-O-, and a structure containing a self-immolative group having a structural formula A shown below.

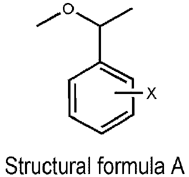

In the above formula, X represents a group that is capable of being changed to an electron-donating group.

The explanation is made below on an example in which X is present at the para-position, but the invention is not limited to this embodiment. When X is changed to an electron-donating group, an elimination reaction proceeds as shown in the scheme below, causing the self-immolative reaction.

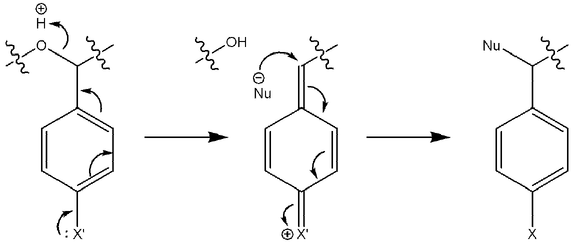

In the above formulae, Nu represents a nucleophile.

With respect to the X, there is no particular limitation, and the group can be appropriately selected from those that have been conventionally known and can be used, and examples of such groups include -NH₂, -OH, -NR₂, -OR, and -R (R represents an alkyl group).

The position of substitution of X to the benzene ring is generally the ortho-position or para-position.

With respect to the stimulus-responsive cleavable macrocyclic tetrazine, specifically, for example, there can be mentioned a compound represented by the following formula.

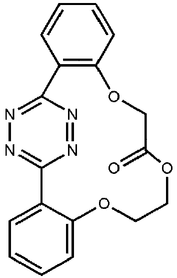

The above-mentioned compound can be cleaved, for example, at pH of 11 to 14, preferably at pH of 13 to 14.

The stimulus-responsive cleavable macrocyclic tetrazine can be produced in accordance with a known organic synthesis method and the description of the Examples below.

Further examples of the stimulus-responsive cleavable macrocyclic tetrazines are shown below, but there is no particular limitation.

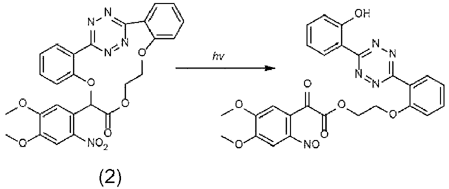

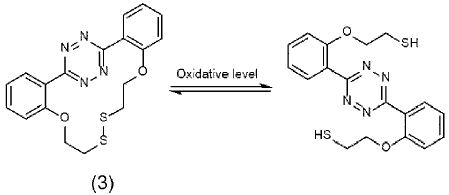

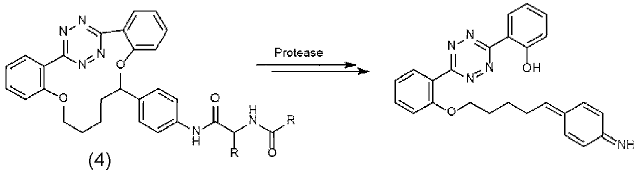

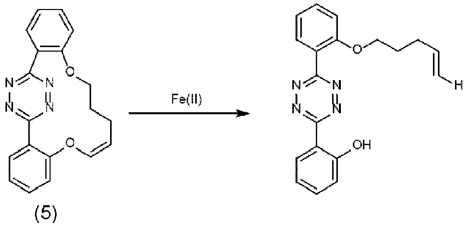

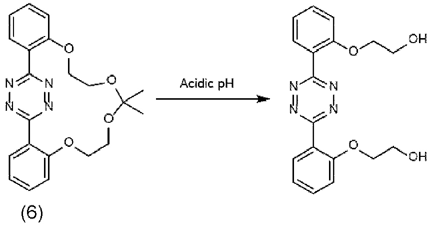

### <Dienophile>

With respect to the dienophile which is a reaction substrate for Diels-Alder reaction, there is no particular limitation as long as the dienophile can undergo a cyclocondensation reaction with a tetrazine, and, according to the purpose of the use of the cyclocondensation product or the like, a dienophile may be appropriately selected from dienophiles which are known reaction substrates for Diels-Alder reaction. Such dienophiles are well-known to those skilled in the art.

The dienophile may be one which has a cyclocondensation reaction moiety with a tetrazine, such as an alkene, an alkyne, a cycloalkene, a cycloalkyne, a heterocycloalkene, or a heterocycloalkyne, and is preferably a cycloalkene, a cycloalkyne, or the like, and more preferred examples include a compound having a structure selected from a trans-cyclooctene, a norbornene, and a cyclooctyne.

In the reaction control method of the invention, a derivative of a dienophile, which is obtained by introducing a substituent, substance or the like, which does not adversely affect the reaction, other than the cyclocondensation reaction moiety with a tetrazine, may be used as the dienophile. An embodiment using such a derivative of dienophile is included in "the use of the dienophile" in the cyclocondensation reaction with a tetrazine.

### <Application to medical and biological technology and the like>

The inverse electron-demand Diels-Alder reaction controlled by the reaction control method of the invention can be applied to, for example, labeling that enables detection of a substance taken into cells, tissues, organs, or the like, analysis of biological intermolecular interaction, analysis of in vivo kinetics of biomolecules, induction of biological intermolecular interaction, drug delivery, in-situ synthesis of medicine, a chemical sensor, and development of an effect, such as a polymerization reaction or gelation reaction in response to a chemical environment. That is, the inverse electron-demand Diels-Alder reaction controlled by the reaction control method of the invention can be applied to medical and biological technology and the like.

For example, when a substituent, substance or the like, which does not adversely affect the reaction, is introduced into the stimulus-responsive cleavable macrocyclic tetrazine and/or dienophile and the inverse electron-demand Diels-Alder reaction is conducted, development of the above-mentioned effect can be achieved. Further, in the invention, the development of the effect can be temporally and quantitatively controlled. With respect to the cleavable macrocyclic tetrazine responsive to a medium that can locally apply a stimulus, such as a light, the development of the effect can be spatially controlled.

With respect to the above-mentioned substituent or substance, there is no particular limitation as long as it does not adversely affect the reaction, and the substituent or substance can be appropriately selected according to the intended function or the like, and may be, for example, a wide range of materials, molecules, or functional portions thereof, which include a polypeptide, an amino acid, a nucleotide, a polynucleotide containing a nucleic acid such as DNA or RNA, biomaterials or biomolecules such as lipid, sugar, and carbohydrate, a surfactant, an enzyme and a substrate, a drug, a ligand substance, a signal transmitter, an agonist, an inhibitor, a labeled material, a gelling material, and a combination thereof. With respect to the substituent or substance that does not adversely affect the reaction, there is no particular limitation, but the substituent or substance may be a drug, a ligand substance, a signal transmitter, an agonist, an inhibitor, a labeled material, a monomer used for polymer synthesis, a gelling material, a combination thereof, or a functional portion thereof. In addition, the substituents or substances which do not adversely affect the reaction may be used individually or in combination arbitrarily with an arbitrary ratio.

With respect to the use, type, and the like of the drug, there is no particular limitation, and examples of drugs include drugs for nervous system and sensory organ (such as a drug for central nervous system, a drug for peripheral nervous system, and a drug for sensory organ), drugs for individual organs (such as a drug for circulatory organ, a drug for respiratory organ, a drug for digestive organ, a hormone agent, and a drug for urogenital organ), metabolic drugs (such as a vitamin compound, a nourishing and tonic medicine, and a drug for blood or lymph), drugs for tissue or cell function (such as an anti-tumor agent, a radiopharmaceutical, and an antiallergic drug), crude drugs, herb medicines, drugs against pathogenic organism (such as antibiotics and a chemotherapeutic agent), a drug for diagnosis, and a drug for xenodiagnosis.

As a ligand substance, there can be mentioned a substance capable of being specifically bonded to a specific protein on a living cell membrane or in a living cell. As a ligand substance, for example, there can be mentioned a substance capable of being specifically bonded to a specific protein to change the function thereof.

With respect to the substance capable of being specifically bonded to a specific protein to change the function thereof, there is no particular limitation, and examples include an extracellular ligand taken into cells from the outside of the cells, and an intracellular ligand produced in cells due to a stimulus from the outside of the cells. For example, the substance may be an agonist or inhibitor (referred to also as antagonist) against a receptor protein (for example, a cell membrane receptor or a nuclear receptor). Further, the substance can be a signal transmitter, such as a signal transmitter protein in cells, or a second messenger.

With respect to the labeled material, there is no particular limitation, and examples of materials include a fluorescence-labeled material, an enzyme-labeled material, and a radioactive material.

Examples of gelling materials include a general natural or synthetic polymer, a copolymer, a living organism-derived polymer, and a composite material thereof, and there is no particular limitation with respect to the gelling material, but, from the viewpoint of excellent biocompatibility and the like, there can be mentioned a gelling material forming a hydrogel, and preferred examples include a fibrin adhesive, collagen, hyaluronic acid, chitosan, gelatin, an alginate, starch, sugar, cellulose, polylactic acid, polyglycolic acid, poly ε-caprolactone, polyethylene glycol, polyacrylic acid, polymethacrylic acid, polyacrylamide, polymethacrylamide, polydimethylmethacrylamide, cyanoacrylate, and derivatives thereof.

Further, when the stimulus-responsive cleavable macrocyclic tetrazine or dienophile used in the reaction control method of the invention is bonded to a carrier, the resultant carrier can be used in isolation of a substance that is specifically bonded to the target protein bonded to the stimulus-responsive cleavable macrocyclic tetrazine or dienophile, purification of a bonded material of the stimulus-responsive cleavable macrocyclic tetrazine or dienophile and the target protein, and the like. With respect to the carrier, there is no particular limitation, and those used in a general biochemical method can be used.

Further, in another embodiment, for example, a signal peptide of a nucleus localization signal (NLS) or the like is introduced into the stimulus-responsive cleavable macrocyclic tetrazine and/or dienophile used in the reaction control method of the invention, and the stimulus-responsive cleavable macrocyclic tetrazine and/or dienophile can be transferred to the intended intracellular organelle. With respect to the signal peptide, there is no particular limitation, and those used in a general biochemical method can be used according to the purpose.

### <Stimulus>

With respect to the stimulus for cleaving the stimulus-responsive cleavable group, there is no particular limitation as long as it can cleave the stimulus-responsive cleavable group, but examples of stimuli include pH, a light, an enzyme, an ion, and oxidation-reduction.

In cleaving the stimulus-responsive cleavable group by a stimulus, the stimulus-responsive group may be a group that is decomposable due to a reduction of the pH value to pH of 6 or less, or may be a group that is decomposable by an increase of the pH value to pH of 10 or more.

With respect to the stimulus-responsive decomposable group that is decomposable due to a change of pH toward the alkaline side, there is no particular limitation, and for example, there can be mentioned a group contained in the compound represented by the formula 1c and a group contained in the compound represented by the formula (6) above.

With respect to the stimulus-responsive decomposable group which is decomposable due to a stimulus of light irradiation, there is no particular limitation, and for example, there can be mentioned a group contained in the compound represented by the formula (2) above.

With respect to the stimulus-responsive decomposable group which is decomposable due to a stimulus caused by an enzyme, there is no particular limitation, and for example, there can be mentioned a group contained in the compound represented by the formula (4) above.

With respect to the stimulus-responsive decomposable group which is decomposable due to a stimulus of an ion, there is no particular limitation, and for example, there can be mentioned a group contained in the compound represented by the formula (5) above.

With respect to the stimulus-responsive decomposable group which is decomposable due to a stimulus of oxidation-reduction, there is no particular limitation, and for example, there can be mentioned a group contained in the compound represented by the formula (3) above.

### <Method for producing a cyclocondensation reaction product>

A cyclocondensation reaction product can be produced by conducting a cyclocondensation reaction of a reaction substrate and a tetrazine using an inverse electron-demand Diels-Alder reaction controlled by the reaction control method of the invention.

Specifically, an embodiment of the invention is directed to a method for producing a cyclocondensation reaction product (hereinafter, referred to also as "the production method of the invention"), which comprises the step of conducting a cyclocondensation reaction of a reaction substrate and a tetrazine using an inverse electron-demand Diels-Alder reaction, in which the reaction control for the inverse electron-demand Diels-Alder reaction is conducted using a stimulus-responsive cleavable macrocyclic tetrazine as the tetrazine.

Further, more specifically, the production method of the invention comprises the following steps a) and b) of:
a) applying a stimulus to cleave a stimulus-responsive cleavable macrocyclic tetrazine; and
b) conducting a cyclocondensation reaction of a reaction substrate and the cleaved macrocyclic tetrazine using an inverse electron-demand Diels-Alder reaction.

Thus, a cyclocondensation reaction of a reaction substrate and a tetrazine is conducted using the controlled inverse electron-demand Diels-Alder reaction, so that a cyclocondensation reaction product can be produced.

The step of cleaving a stimulus-responsive cleavable macrocyclic tetrazine and the step of conducting a cyclocondensation reaction of a reaction substrate and a tetrazine using the cleaved macrocyclic tetrazine in an inverse electron-demand Diels-Alder reaction are similar to those described above in connection with <the reaction control method of the invention>.

That is, all the description mentioned above in connection with <the reaction control method of the invention> is applied to the description of the production method of the invention.

Further, another embodiment of the invention is directed to a method for conducting a cyclocondensation reaction, which comprises the step of conducting a cyclocondensation reaction of a reaction substrate and a tetrazine using an inverse electron-demand Diels-Alder reaction, in which the reaction control for the inverse electron-demand Diels-Alder reaction is conducted using a stimulus-responsive cleavable macrocyclic tetrazine as the tetrazine.

All the description mentioned above in connection with <the reaction control method of the invention> is applied to the description of the method for conducting a cyclocondensation reaction.

### <Compound>

An embodiment of the invention is directed to a compound represented by the following formula (I) (hereinafter, frequently referred to as "the compound of the invention"): 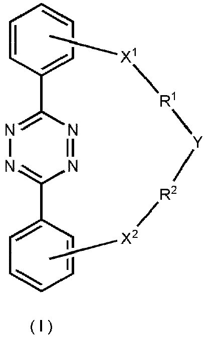
in which X¹ and X² are a heteroatom or a carbon atom,
R¹ and R² are independently a single bond, an alkylene, a heteroalkylene, an arylene, a heteroarylene, -CO-, or a combination thereof, and Y is a stimulus-responsive cleavable group.

Specific examples of X¹, X², R¹, R², and Y and the like are the same as those described above in connection with <the reaction control method of the invention>.

The compound of the invention may introduce a substituent, substance or the like, which does not adversely affect the reaction, and may be derivatized, then used in the reaction control method of the invention and/or the production method of the invention. Introduction of a substituent, substance or the like, which does not adversely affect the reaction, into the compound of the invention can be performed using a known organic synthesis method.

According to an embodiment of the invention, in the compound represented by the formula (I), X¹ and X² are oxygen.

According to another embodiment of the invention, in the compound represented by the formula (I), R¹ and R² are independently a single bond, an alkylene, an arylene, or a combination thereof.

According to further another embodiment of the invention, in the compound represented by the formula (I), the stimulus-responsive cleavable group is selected from groups represented by -COO-, -O-, -S-S-, or -O-C(CH₃)₂-O-.

According to an embodiment of the invention, in the compound represented by the formula (I), X¹ and X² are oxygen, R¹ and R² are independently a single bond, an alkylene, an arylene, or a combination thereof, and the stimulus-responsive cleavable group is selected from groups represented by -COO-, -O-, -S-S-, or -O-C(CH₃)₂-O-.

According to another embodiment of the invention, the compound represented by the formula (I) is a compound represented by the following formula. The below-shown compound corresponds to the compound 1c described in the Examples below.

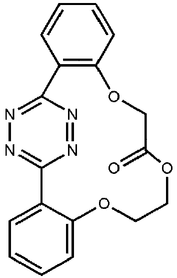

The compound of the invention can be produced in accordance with a known organic synthesis method and the description of the Examples below.

The compound of the invention is a novel compound, and is cleavable in response to a stimulus such as pH. Therefore, the compound of the invention can be used as a stimulus-responsive cleavable macrocyclic tetrazine in, for example, the reaction control method of the invention or the production method of the invention.

Note that all the description mentioned above in connection with <the reaction control method of the invention> and <the production method of the invention> is applied to the description of the compound of the invention.

### <Reagent>

An embodiment of the invention is directed to a reagent for an inverse electron-demand Diels-Alder reaction, which comprises the compound of the invention (hereinafter, frequently referred to as "the reagent of the invention").

As mentioned above, the compound of the invention has specific and high reactivity with a dienophile in an inverse electron-demand Diels-Alder reaction, and therefore, using bonding between the compound of the invention and a dienophile, the compound can be used as a reagent for protein labeling, analysis of biological intermolecular interaction, analysis of in vivo kinetics of biomolecules, induction of biological intermolecular interaction, or the like. In the reagent of the invention, the compound of the invention can be used alone or in combination of two or more types.

The reagent of the invention is described below with reference to an embodiment as an example in which a ligand compound and/or a protein to be bonded to a ligand compound is bonded to the compound of the invention, and a target protein is bonded to a dienophile, but the invention is not limited to this embodiment.

The compound of the invention may be contained in the reagent in a form such that the compound of the invention and a ligand compound and/or a protein to be bonded to a ligand compound, or the like are bonded, or may be in a form such that the compound of the invention is contained in the reagent in the form of the compound of the invention and, when using the reagent, the compound is bonded to a ligand compound and/or a protein to be bonded to a ligand compound, or the like. In addition, the protein to be bonded to a ligand compound may be contained in the reagent in the form of a DNA such as a vector containing expressably the protein to be bonded to a ligand compound.

Further, the dienophile may be contained in the reagent in a form such that the dienophile and a target protein or the like are bonded, or may be in a form such that the dienophile is contained in the reagent in the form of the dienophile and, when using the reagent, the dienophile is bonded to a target protein or the like. In addition, the target protein may be contained in the reagent in the form of a DNA such as a vector containing expressably the target protein.

The reagent of the invention may be used in the form of a reagent kit, and may contain, in addition to the compound of the invention, one or two or more reagents that can be used as a reagent for a dienophile, labeling or analysis of biological intermolecular interaction, analysis of in vivo kinetics of biomolecules, induction of biological intermolecular interaction, or the like. Examples of such reagents include a dienophile, a reagent for a Diels-Alder reaction, a buffer, a preservative, or a stabilizing agent for stably maintaining the compound of the invention and dienophile, a reagent for bonding the compound of the invention and a ligand compound to form a derivative, a reagent for bonding a dienophile and a target protein to form a derivative, a separation reagent, a labeled material, and an expression vector (which generally contains at least one member of a promoter gene, a restriction enzyme site for inserting a target protein gene, a target protein gene, a selective marker, a gene of a protein to be bonded to a ligand compound, and the like), but there is no particular limitation with respect to the reagent.

### Examples

Hereinbelow, the invention will be described in detail with reference to the following Examples, which are examples of the invention and should not be construed as limiting the scope of the invention.

### <Synthesis Example>

The compounds (macrocyclic tetrazines 1a to 1e and noncyclic tetrazine 2) used in the Examples are shown below.

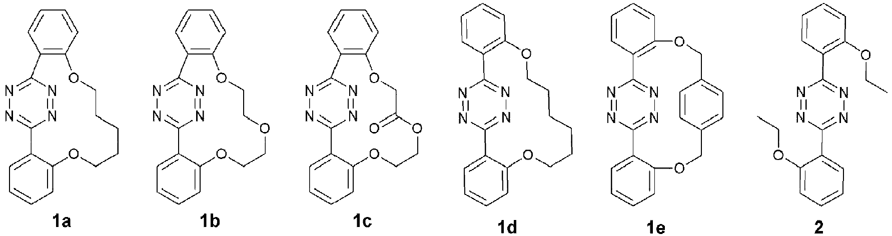

The macrocyclic tetrazines and noncyclic tetrazine were synthesized as described below. The compounds 4 and 5 were synthesized in accordance with the method described in the reference documents (S. Patra, et al., "Controlling Metal-Ligand-Metal Oxidation State Combinations by Ancillary Ligand (L) Variation in the Redox Systems [L2Ru (µ-boptz)RuL2]n, boptz=3,6-bis(2-oxidophenyl)-1,2,4,5-tetrazine, and L=acetylacetonate, 2,2'-bipyridine, or 2-phenylazopyridine", Chem. Eur. J. 12, 489-498 (2006).; and A. Sergey et al., "Experimentally realized mechanochemistry distinct from force-accelerated scission of loaded bonds", Science 357, 299-303 (2017)).

A synthesis scheme is shown below.

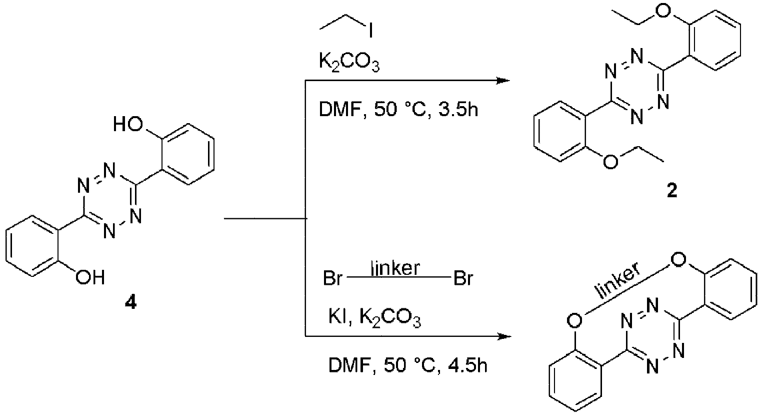

### <Synthesis of compound 3,6-bis(2-ethoxyphenyl)-1,2,4,5-tetrazine (2)>

The compound 4 (10 mg, 0.038 mmol, 1 eq), K₂CO₃ (16.2 mg, 0.12 mmol, 3 eq), and ethyl iodide (10.0 µL, 0.12 mmol, 3 eq) solution (in 5 mL of dry DMF) were stirred in an argon atmosphere at 50°C for 4.5 hours. The resultant reaction mixture was cooled to 0°C, and quenched with 20 mL of HCl 0.5 M, and diluted with 30 mL of Et₂O. The reaction mixture was subjected to extraction with Et₂O (2 × 30 mL), and the organic layers were combined and washed with 25 mL of brine, and dried over Na₂SO₄, and concentrated under a reduced pressure. The crude product was purified by silica gel column chromatography using DCM, obtaining 10.6 mg of compound 2 in the form of a pink solid (yield: 44%).

¹H NMR (400 MHz, CDCl₃): δ 8.03 (dd, 1H, J = 7.6, 1.7 Hz), 7.55 (ddd, 1H, J = 8.4, 7.5, 1.7 Hz), 7.17 (td, 1H, J = 7.5, 1 Hz), 7.12 (d, 1H, J = 8.4 Hz), 4.19 (q, 2H, J = 7.0 Hz), 1.40 (t, 3H, J = 7.0 Hz).
¹³C NMR (100 MHz, CDCl₃) : δ 164.64, 158.06, 133.13, 131.96, 122.71, 121.13, 113.58, 64.95, 14.80.
HRMS m/z (ESI) : calcd. for C₁₈H₁₈N₄O₂ [M+H]⁺: 323.1503; found: 323.1503.

### <General synthesis method for macrocyclic tetrazine>

0.5 mM diphenoltetrazine 4 (1.0 eq), K₂CO₃ (3.0 eq), KI (3.0 eq), and a dibromoalkane (1.1 eq) (in 75 mL of dry DMF) were stirred in an argon atmosphere at 50°C for 4.5 hours. The resultant reaction mixture was cooled to 0°C, and quenched with 20 mL of HCl 0.5 M, and 25 mL of brine was added. The reaction mixture was subjected to extraction with Et₂O (2 × 50 mL), and the organic layers were combined and washed with water (2 × 25 mL), and then washed with 25 mL of brine, and dried over Na₂SO₄, and concentrated under a reduced pressure. The crude product was purified by silica gel column chromatography and/or HPLC.

### <Synthesis of compound 4,10-dioxa-2(3,6)-tetrazina-1,3(1,2)-dibenzenacyclodecaphane (1a)>

A synthesis scheme is shown below.

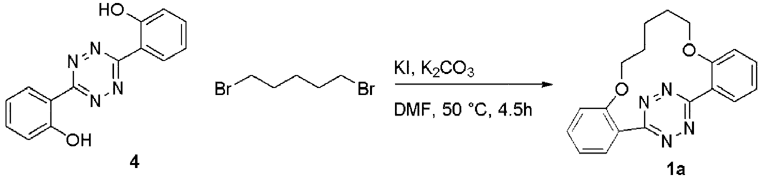

A general synthesis method for macrocyclic tetrazine was conducted using the tetrazine 4 (21.3 mg, 0.080 mmol, 1 eq), K₂CO₃ (32.6 mg, 0.24 mmol, 3 eq), KI (39.6 mg, 0.24 mmol, 3 eq), and 1,5-dibromopentane (12.5 µL, 0.093 mmol, 1 eq). Purification was made by silica gel column chromatography using DCM and RP-HPLC (gradient: 50 - 90% 0.1% HCOOH in H₂O/0.1% HCOOH in MeCN), obtaining 6.5 mg of compound 1a in the form of an orange solid (yield: 24%).

¹H NMR (400 MHz, CDCl₃): δ 8.16 (dd, 1H, J = 7.6, 2 Hz), 7.55 (ddd, 1H, J = 8.3, 7.5, 2 Hz), 7.22 (td, 1H, J = 7.5, 1.0 Hz), 7.05 (dd, 1H, J = 8.3, 1.0 Hz), 3.94 (t, 2H, J = 5.3 Hz), 1.71 (m, 2H), 1.22 (m, 1H).
¹³C NMR (100 MHz, CDCl₃) : δ 161.69, 157.71, 133.20, 130.00, 123.01, 121.43, 113.74, 68.16, 29.51, 21.20.
HRMS m/z (ESI) : calcd. for C₁₉H₁₈N₄O₂ [M+H]⁺: 335.1503; found: 335.1503.

### <Synthesis of compound 4,7,10-trioxa-2(3,6)-tetrazina-1,3(1,2)-dibenzenacyclodecaphane (1b)>

A synthesis scheme is shown below.

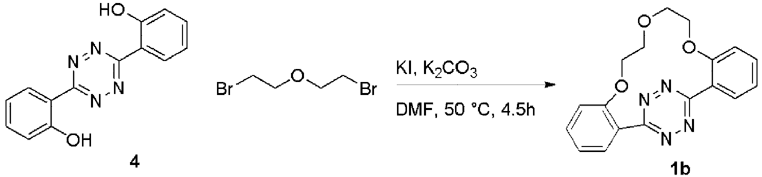

A general synthesis method for macrocyclic tetrazine was conducted using the tetrazine 4 (21.3 mg, 0.081 mmol, 1 eq), K₂CO₃ (33.0 mg, 0.24 mmol, 3 eq), KI (39.8 mg, 0.24 mmol, 3 eq), and 2-bromoethyl ether (11.0 µL, 0.087 mmol, 1 eq). Purification was made by a silica gel column using DCM containing 20% EtOAc, obtaining 2.6 mg of compound 1b in the form of an orange solid (yield: 10%).

¹H NMR (400 MHz, CDCl₃): δ 8.18 (dd, 1H, J = 7.6, 1.7 Hz), 8.29 (ddd, 1H, J = 8.3, 7.5, 1.7 Hz), 7.54 (td, 1H, J = 7.6, 1.0 Hz), 7.06 (dd, 1H, J = 8.3, 1.0 Hz), 4.08-3.73 (m, 4H). ¹³C NMR (100 MHz, CDCl₃) : δ 161.22, 157.47, 133.08, 130.03, 123.89, 122.20, 114.86, 70.12, 68.93.
HRMS m/z (ESI) : calcd. for C₁₈H₁₇N₄O₃ [M+H]⁺: 337.1295; found: 336.1295.

### <Synthesis of compound 4,7,10-trioxa-2(3,6)-tetrazina-1,3(1,2)-dibenzenacyclodecaphan-6-one (1c)>

A synthesis scheme is shown below.

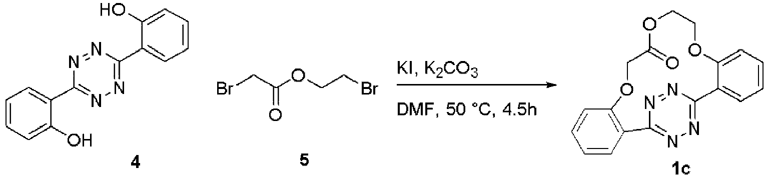

A general synthesis method for macrocyclic tetrazine was conducted using the tetrazine 4 (20.8 mg, 0.078 mmol, 1 eq), K₂CO₃ (33.9 mg, 0.24 mmol, 3 eq), KI (39.8 mg, 0.24 mmol, 3 eq), and the dibromoalkane 5 (21.2, 0.086 mmol, 1.2 eq). Purification was made by a silica gel column using EtOAc and RP-HPLC (gradient: 50 - 90% 0.1% HCOOH in H₂O/0.1% HCOOH in MeCN), obtaining 10.1 mg of compound 1c in the form of an orange solid (yield: 37%).

¹H NMR (400 MHz, CDCl₃): δ 8.18 (m, 2H), 7.67 (m, 2H), 7.37 (td, 1H, J = 7.6, 1.0 Hz), 7.31 (td, 1H, J = 7.6, 1.0 Hz), 7.26 (dd, 1H, J = 8.3, 1.0 Hz), 7.00 (dd, 1H, J = 8.3, 1.0 Hz), 4.62 (s, 2H), 4.45-4.25 (m, 4H).
¹³C NMR (100 MHz, CDCl₃) : δ 167.95, 161.87, 161.79, 157.19, 156.08, 133.35, 133.17, 130.28, 130.05, 126.09, 124.05, 123.64, 122.78, 119.19, 113.83, 70.01, 67.10, 64.30.
HRMS m/z (ESI) : calcd. for C₁₈H₁₄N₄O₄ [M+H]⁺: 351.1088; found: 351.1088.

### <Synthesis of compound 4,11-dioxa-2(3,6)-tetrazina-1,3(1,2)-dibenzenacycloundecaphane (1d)>

A synthesis scheme is shown below.

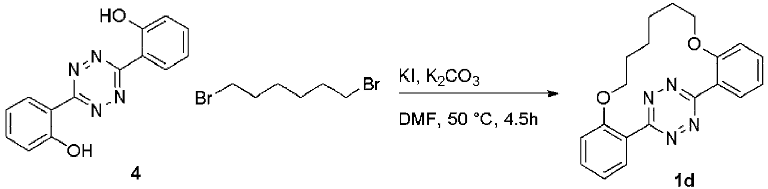

A general synthesis method for macrocyclic tetrazine was conducted using the tetrazine 4 (20.4 mg, 0.077 mmol, 1.0 eq), K₂CO₃ (32.6 mg, 0.24 mmol, 3 eq), KI (37.7 mg, 0.23 mmol, 3 eq), and 1,6-dibromohexane (12.5 µL, 0.081 mmol, 1 eq). Purification was made by a silica gel column using DCM and RP-HPLC (gradient: 50 - 90% 0.1% HCOOH in H₂O/0.1% HCOOH in MeCN), obtaining 8.5 mg of compound 1d in the form of a red solid (yield: 310).

¹H NMR (400 MHz, CDCl₃): δ 8.08 (dd, 1H, J = 7.6, 2 Hz), 7.56 (ddd, 1H, J = 8.4, 7.5, 2 Hz), 7.20 (td, 1H, J = 7.5, 1 Hz), 7.06 (d, 1H, J = 8.4 Hz), 4.03 (t, 2H, J = 5.3 Hz), 1.72 (m, 2H), 1.38 (m, 2H).
¹³C NMR (100 MHz, CDCl₃): δ 163.29, 1578.10, 133.12, 130.53, 122.90, 121.20, 113.20, 70.26, 29.23, 28.26.
HRMS m/z (ESI) : calcd. for C₂₀H₂₀N₄O₂ [M+H]⁺: 349.1659; found: 349.1659.

### <Synthesis of compound 4,8-dioxa-2(3,6)-tetrazina-1,3(1,2),6(1,4)-tribenzenacyclooctaphane (1e)>

A synthesis scheme is shown below.

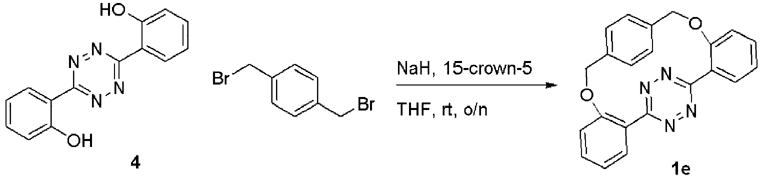

A general synthesis method for macrocyclic tetrazine was conducted using the tetrazine 4 (20.8 mg, 0.078 mmol, 1 eq), K₂CO₃ (31.5 mg, 0.23 mmol, 3 eq), KI (37.6 mg, 0.23 mmol, 3 eq), and α,α'-dibromo-p-xylene (22.8, 0.086 mmol, 1 eq). Purification was made by a silica gel column using DCM, obtaining 14.5 mg of compound 1e in the form of a pink crystal (yield: 50%).

¹H NMR (400 MHz, CDCl₃): δ 8.22 (dd, 2H, J = 7.7, 1.8 Hz), 7.73 (ddd, 2H, J = 8.2, 7.3, 1.8 Hz), 7.44 (m, 4H), 6.76 (s, 4H), 4.32 (s, 4H).
¹³C NMR (100 MHz, CDCl₃): δ 162.57, 157.88, 135.24, 134.25, 130.97, 129.82, 128.66, 124.97, 124.32, 79.88.
HRMS m/z (ESI) : calcd. for C₂₂H₁₆N₄O₂ [M+H]⁺: 369.1346; found: 369.1346.

### <Analysis of reactivity by HPLC>

50 µL of the reaction mixture was diluted with water containing 0.1% HCOOH so that the amount of the diluted mixture became 300 µL, and 100 µL of the diluted reaction mixture was subjected to analysis by RP-HPLC (5 - 70% 0.1% HCOOH in MeCN/0.1% HCOOH in H₂O; monitored for 30 minutes or more; flow rate: 1 mL/min). New peaks were analyzed by mass spectrometry.

### <Example 1> Reaction of Tz and TCO (absorption spectrum analysis)

The effect of a change of the tetrazine (Tz) to macrocyclic structure on the IEDDA reaction was evaluated.

Specifically, with respect to the macrocyclic tetrazines 1a to 1e and noncyclic tetrazine 2, the reactivity with TCO-Morph was evaluated. A reaction scheme of Tz and TCO-Morph is shown below.

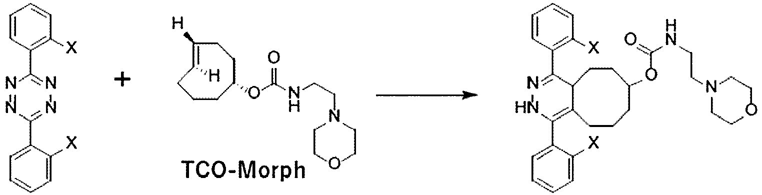

Each of the macrocyclic tetrazines and noncyclic tetrazine was added to MeCN so that the final concentration became 0.1 mM, and absorption spectra were measured. 2 mM TCO-Morph was added, and the resultant mixture was subjected to reaction at 25°C for one hour, and absorption spectra were measured at intervals of one minute.

With respect to the macrocyclic tetrazines 1a to 1e, no change of the absorbance was seen after adding TCO-Morph, and it was found that a cyclocondensation reaction did not proceed (a to e of FIG. 1, and FIG. 2). On the other hand, with respect to the noncyclic tetrazine 2, the absorbance was drastically reduced after adding TCO-Morph, and it is considered that a cyclocondensation reaction proceeded (f of FIG. 1, and FIG. 2). From this, it was found that a tetrazine having a macrocyclic structure enables termination of the reaction activity of the tetrazine.

### <Example 2> Reaction of Tz and TCO (HPLC analysis)

With respect to each of the macrocyclic tetrazines 1a to 1e and noncyclic tetrazine 2, the reaction product with TCO-Morph was analyzed by HPLC.

With respect to the macrocyclic tetrazines 1a to 1e, after adding TCO-Morph (after one hour and 24 hours), only a peak derived from the macrocyclic tetrazine was obtained, and the HPLC analysis also showed that a cyclocondensation reaction did not proceed even after a reaction for a long time (a to e of FIG. 3). On the other hand, with respect to the noncyclic tetrazine 2, after adding TCO-Morph (after one minute and one hour), a peak derived from the noncyclic tetrazine drastically decreased, and a peak derived from the cyclocondensation product increased, and the results showed that a cyclocondensation reaction proceeded in a short time (f of FIG. 3). From this, it was found that a tetrazine having a macrocyclic structure enables termination of the reaction activity of the tetrazine, and that a tetrazine having a noncyclic structure enables development of the reaction activity of the tetrazine.

<Example 3> Stimulus-responsive IEDDA reaction

The macrocyclic tetrazine 1c is cleaved in response to alkaline (high pH) conditions to form a noncyclic tetrazine 2c (a of FIG. 4).

With respect to the macrocyclic tetrazine 1c, the reactivity with TCO-Morph for stimulus-responsive IEDDA reaction was evaluated. The macrocyclic tetrazine 1c was added and TCO-Morph was added (or not added) to 1:1 MeCN/aqueous NaOH solution (1.4% DMSO) (500 µL in total) so that the final concentration of the macrocyclic tetrazine 1c became 0.1 mM and the final concentration of TCO-Morph became 0.6 mM, and the resultant mixture was subjected to reaction at 25°C for 10 minutes, and analyzed the reaction product by HPLC.

In the system of 1:1 MeCN/2 mM aqueous NaOH solution (b of FIG. 4), in the case of adding no TCO-Morph, only a peak derived from the macrocyclic tetrazine 2c was found. In the case of adding TCO-Morph, a peak derived from the macrocyclic tetrazine 2c was not found, and only a peak derived from the Pdz-c which is a cyclocondensation product was found. The results showed that the macrocyclic tetrazine 1c was cleaved due to a pH stimulus to form the noncyclic tetrazine 2c, which reacted with TCO-Morph. On the other hand, in the system of 1:1 MeCN/1 mM aqueous NaOH solution (c of FIG. 4), in the case of adding no TCO-Morph, a peak derived from the macrocyclic tetrazine 1c and a peak derived from the noncyclic tetrazine 2c were found. It was found that part of the macrocyclic tetrazine 1c was not cleaved. Further, in the case of adding TCO-Morph, a peak derived from the macrocyclic tetrazine 1c and a peak derived from the Pdz-c were found. The results showed that the macrocyclic tetrazine 1c was cleaved due to a pH stimulus to form the noncyclic tetrazine 2c, which reacted with TCO-Morph, but part of the macrocyclic tetrazine 1c was not cleaved and remained intact. From this, it was found that, by using the macrocyclic tetrazine, the IEDDA reaction, including the degree of the reaction, can be controlled by a stimulus.

### <Example 4> Stimulus-responsive IEDDA reaction

With respect to the macrocyclic tetrazines 1a and 1c and noncyclic tetrazine 2, the states under neutral conditions and alkaline conditions were evaluated. Each of the macrocyclic tetrazines and noncyclic tetrazine was added to 1:1 MeCN/100 mM HEPES pH 7.4 or a 2 mM aqueous NaOH solution (1.4% DMSO) (500 µL in total) so that the final concentration became 0.1 mM, and the resultant mixture was incubated at 25°C for 10 minutes, and absorption spectra were measured. 0.4 mM TCO-Morph was added, and the resultant mixture was subjected to reaction at 25°C for one hour, and absorption spectra were measured at intervals of one minute.

With respect to the macrocyclic tetrazine 1a and noncyclic tetrazine 2, almost no change was found in the absorption spectrum and absorption maximum wavelength under neutral conditions and alkaline conditions, and it was found that the structure of the tetrazine was not changed. On the other hand, with respect to the macrocyclic tetrazine 1c, a change was found in the absorption spectrum and absorption maximum wavelength under neutral conditions and alkaline conditions, and it was found that the structure of the tetrazine was changed (a and b of FIG. 5). Further, after adding TCO-Morph, almost no change with time was seen in the absorbance of the macrocyclic tetrazines 1a and 1c under neutral conditions, whereas the absorbance of the noncyclic tetrazine 2 under neutral conditions was reduced in a short time (c of FIG. 5). It was found that only a cyclocondensation reaction of the noncyclic tetrazine 2 with TCO-Morph proceeded under neutral conditions. After adding TCO-Morph, almost no change with time was seen in the absorbance of the macrocyclic tetrazine 1a under alkaline conditions, whereas the absorbance of the macrocyclic tetrazine 1c and noncyclic tetrazine 2 under alkaline conditions was reduced in a short time (d of FIG. 5). It was found that the macrocyclic tetrazine 1c was cleaved under alkaline conditions and the tetrazine became reactive, so that a cyclocondensation reaction with TCO-Morph proceeded. From this, it was found that, by using the stimulus-responsive macrocyclic tetrazine, the IEDDA reaction can be controlled by a stimulus.

### Industrial Applicability

The invention can be used in the fields of medicine, biology, and the like.

## Claims

1. A method for producing a cyclocondensation reaction product, comprising the step of conducting a cyclocondensation reaction of a reaction substrate and a tetrazine using an inverse electron-demand Diels-Alder reaction,
wherein the reaction control for the inverse electron-demand Diels-Alder reaction is conducted using a stimulus-responsive cleavable macrocyclic tetrazine as the tetrazine.

2. The production method according to claim 1, which comprises the following steps a) and b) of:
a) applying a stimulus to cleave a stimulus-responsive cleavable macrocyclic tetrazine; and
b) conducting a cyclocondensation reaction of a reaction substrate and the cleaved macrocyclic tetrazine using an inverse electron-demand Diels-Alder reaction.

3. The production method according to claim 1 or 2, wherein the stimulus-responsive cleavable macrocyclic tetrazine is a compound having a cyclic moiety linking two carbon atoms of the tetrazine, and having a stimulus-responsive cleavable group in the cyclic moiety.

4. The production method according to any one of claims 1 to 3, wherein the stimulus is selected from pH, a light, an enzyme, an ion, and oxidation-reduction.

5. The production method according to any one of claims 1 to 4, wherein the tetrazine is 1,2,4,5-tetrazine.

6. The production method according to any one of claims 1 to 5, wherein the stimulus-responsive cleavable macrocyclic tetrazine is a compound represented by the following formula (I): 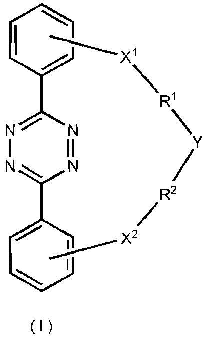
wherein X¹ and X² are a heteroatom or a carbon atom,
R¹ and R² are independently a single bond, an alkylene, a heteroalkylene, an arylene, a heteroarylene, -CO-, or a combination thereof, and Y is a stimulus-responsive cleavable group.

7. The production method according to any one of claims 1 to 6, wherein the stimulus-responsive cleavable macrocyclic tetrazine is a compound represented by the following formula:

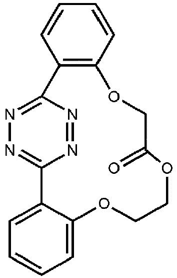

8. The production method according to any one of claims 1 to 7, wherein the reaction substrate is a compound having a structure selected from a transcyclooctene, a norbornene, and a cyclooctyne.

9. A reaction control method for an inverse electron-demand Diels-Alder reaction, comprising the step of conducting, in a cyclocondensation reaction of a reaction substrate and a tetrazine using an inverse electron-demand Diels-Alder reaction, the reaction control for the inverse electron-demand Diels-Alder reaction using a stimulus-responsive cleavable macrocyclic tetrazine as the tetrazine.

10. A compound represented by the following formula (I): 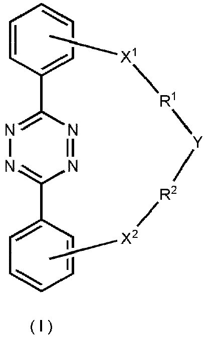
wherein X¹ and X² are a heteroatom or a carbon atom,
R¹ and R² are independently a single bond, an alkylene, a heteroalkylene, an arylene, a heteroarylene, -CO-, or a combination thereof, and Y is a stimulus-responsive cleavable group.

11. The compound according to claim 10, wherein the X¹ and X² are oxygen.

12. The compound according to claim 10 or 11, wherein the R¹ and R² are independently a single bond, an alkylene, an arylene, or a combination thereof.

13. The compound according to any one of claims 10 to 12, wherein the stimulus-responsive cleavable group is selected from -COO-, -CONH-, -O-, -S-S-, -O-C(CH₃)₂-O-, -O-PO(O-)-O-, and a structure containing a self-immolative group having a structural formula A shown below: 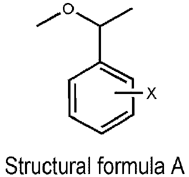 wherein X represents a group which is capable of being changed to an electron-donating group.

14. The compound according to any one of claims 10 to 13, which is a compound represented by the following formula:

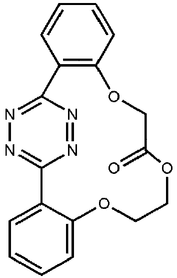

15. A reagent for an inverse electron-demand Diels-Alder reaction, comprising the compound according to any one of claims 10 to 14.
